(19) Europäisches Patentamt / European Patent Office / Office européen des brevets

(11) **EP 2 811 012 A1**

(12) **EUROPEAN PATENT APPLICATION**

(43) Date of publication:
**10.12.2014 Bulletin 2014/50**

(51) Int Cl.:
*C12M 3/06* *(2006.01)*     *C12M 1/12* *(2006.01)*

(21) Application number: **13171049.3**

(22) Date of filing: **07.06.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Eidgenossisch Technische Hochschule Zürich**
**8092 Zürich (CH)**

(72) Inventor: **Frey, Olivier**
**4052 Basel (CH)**

(74) Representative: **Stolmár & Partner**
**Blumenstraße 17**
**80331 München (DE)**

(54) **Hanging network plate**

(57) A hanging network plate is provided, comprising a network surface including at least two hanging drop compartments and at least one network channel fluidly connecting the hanging drop compartments, wherein the at least two hanging drop compartments and the at least one network channel are surface patterns confined by at least one retention structure. Further provided are a use of the hanging network plate and a method of producing a hanging liquid network.

FIG 1

EP 2 811 012 A1

## Description

The present invention is directed to a hanging network plate, a method for producing a hanging network plate, a use of the hanging network plate and a method of producing a hanging liquid network.

In-vitro cell-based assays are a pivotal element in the overall drug development process. At an early time point, they provide essential information on the efficacy and toxicity of a newly discovered compound and help to decide, whether the candidate drug should be further developed or already abandoned. Therefore, there is a large interest that the information obtained in such assays is highly significant, and correctly predicts the biochemical impact of compounds on specific organs or the whole human body. The widely used 2D cell cultures are continuously improved in order to better mimic the complex processes observed in-vivo and have been adopted for massively parallel screening. They, however, still show limitations and are not able to represent an important set of morphological, mechanical and biochemical properties.

This motivates the strong present effort to develop three-dimensional tissue structures, as they have been proven to exhibit functionalities, which closer resemble those of a living organism. Many approaches for producing and culturing 3D tissues are currently developed or are already available spanning a wide range of complexities, see for instance Pampaloni, F. and Stelzer, E. Three-dimensional cell cultures in toxicology. Biotechnology & genetic engineering reviews 26, 117-38 (2010); and Lee, J., Cuddihy, M. J. & Kotov, N. Three-dimensional cell culture matrices: state of the art. Tissue engineering. Part B, Reviews 14, 61-86 (2008). Beside rather conventional cell culture techniques and set-ups, based on well-plates, microfabricated devices become more and more popular, such as disclosed by Khademhosseini, A., Langer, R., Borenstein, J. & Vacanti, J. P. Microscale technologies for tissue engineering and biology; Proceedings of the National Academy of Sciences of the United States of America 103, 2480-7 (2006). On the one hand they allow for manipulating cells in microstructures for tissue assembly, and, on the other hand, they can provide precisely controllable microenvironments that are similar to in-vivo conditions with respect to tissue-to-media ratios, fluid residence times and dynamic mechanical forces (Huh, D., Torisawa, Y., Hamilton, G. a, Kim, H. J. & Ingber, D. E. Microengineered physiological biomimicry: organs-on-chips. Lab on a chip 12, 2156-64 (2012)).

Designing in-vitro systems for predictive assays is a trade-off between, on the one hand, complexity and high integration to represent the specific human organ as closely as possible and extract highly relevant information and, on the other hand, simplicity for straightforward handling and the suitability for parallelization and related high-throughput screening. In general, a system has to be as simple as possible and as complex as needed with relation to the targeted data quality.

Spherical microtissues (MT) are scaffold-free cell clusters in the diameter range between 100 to 500 $\mu$m expressing tissue-like phenotype and functionality (Friedrich, J., Ebner, R. & Kunz-Schughart, L. a Experimental anti-tumor therapy in 3-D: spheroids - old hat or new challenge? International journal of radiation biology 83, 849-71 (2007); Hirschhaeuser, F. et al. Multicellular tumor spheroids: an underestimated tool is catching up again. Journal of biotechnology 148, 3-15 (2010)).

The small size and compact 3D-structure of MTs make them easy to handle and compatible with array-based formats. Spherical microtissues can be generated using the hanging drop technique: In its simplest form, a droplet of specific cell concentration is pipetted on a flat surface and flipped upside down. The suspended cells sediment due to gravity to the liquid-air interface and aggregate into spheroids forming their own extracellular matrix (Kelm, J. M. & Fussenegger, M. Microscale tissue engineering using gravity-enforced cell assembly. Trends in biotechnology 22, 195-202 (2004)). This rather exploratory technique has been replaced recently by micro-titer plates with direct access to the droplets from the top, which obviate substrate flipping and allow for precise pipetting and media exchange via automated systems (Hsiao, A. Y. et al. Micro-ring structures stabilize microdroplets to enable long term spheroid culture in 384 hanging drop array plates. Biomedical microdevices 14, 313-23 (2012); Drewitz, M. et al. Towards automated production and drug sensitivity testing using scaffold-free spherical tumor microtissues. Biotechnology journal 6, 1488-96 (2011)). Thereby, a highly parallel-ized MT fabrication at high reproducibility can be obtained. However, there is still a demand for providing improved tissue production and culturing devices.

Object of the invention is to provide a device and a method for producing and culturing cells and tissue.

This object is achieved by a hanging network plate according to claim 1, a method for producing a hanging network plate according to claim 11, a use of the hanging network plate according to claim 13, and a method of producing a hanging liquid network according to claim 14.

In a first embodiment, a hanging network plate is provided, including a network surface including at least two hanging drop compartments and at least one network channel fluidly connecting the hanging drop compartments, wherein the at least two hanging drop compartments and the at least one network channel are surface patterns confined by at least one retention structure.

According to a second embodiment, a method for producing a hanging network plate of the first embodiment is provided, including forming a hanging network plate having a network surface including at least two hanging drop compartments and at least one network channel fluidly connecting the hanging drop compartments, wherein the at least two hanging drop compartments and the at least one network channel are surface patterns confined by at least one retention structure; and/or forming a negative mold of the hanging network plate and casting the material of the hanging network plate in the mold.

A third embodiment is directed to a use of the hanging network plate of the first embodiment for producing and/or culturing cells or tissue, for engineering tissue, for forming spherical microtissue, for performing assays, for performing in-vitro cell-based assays, for performing toxicity assays, and/or for performing protein crystallization.

In a fourth embodiment, a method for producing a hanging liquid network including liquid drops and/or a liquid network is provided, including arranging the hanging network plate of the first embodiment such that the network surface is positioned at the bottom of the hanging network plate.

Surprisingly, embodiments of the invention allow for providing a fully interconnected hanging liquid network, in particular a microfluidic network. The hanging drops are acting as small liquid spaces that form and host suspended and/or dissolved matter, for instance microtissue, and are interconnected with hanging liquid conduits surprisingly allowing for continuously exchanging matter, e.g. a continuous nutrient perfusion and metabolite exchange. With regard to in-vitro cell-based assays the embodiments of the invention not only enable more complex studies with 3D tissue models, but also promote tissue production and culturing.

While the foregoing and the following is directed to examples and embodiments of the invention, other and further embodiments of the invention may be devised. Especially, mutually non-exclusive features of the examples and embodiments of the invention may be combined with each other. Some of the above mentioned embodiments will be described in more detail in the following description of typical embodiments with reference to the following drawings in which:

Fig. 1               schematically illustrates a plan view of a hanging network plate according to an embodiment of the invention;

Fig. 2a and 2b       schematically illustrate a partial bottom plan view and a partial side view of the embodiment of Fig. 1 provided with a hanging liquid network;

Figs. 3a to 3d       schematically show a cross sectional view of the network channel according to embodiments of the invention;

Figs. 4a and 4b      schematically illustrate a cross sectional view of the hanging drop compartment of embodiments of the invention;

Figs. 5a to 5f       schematically illustrate examples of the hanging network plate according to embodiments of the invention, Fig. 5b further showing a diagram relating to an experiment;

Figs. 6a and 6b      schematically illustrate further examples of the hanging network plate according to embodiments of the invention;

Figs. 7A to 7C       are microscope images showing the results of a bead distribution test;

Figs. 8A and 8B      are microscope images showing the results of a parallel microtissue formation test;

Fig. 9               presents a series of microscope images showing the results of a continuous microtissue perfusion test; and

Fig. 10              schematically shows a part of a negative mold for forming the hanging network plate according to an example.

Within the following description of the drawings, the same reference numbers refer to the same components. Generally, only the differences with respect to the individual embodiments are described. In the following description, embodiments of the invention are described referring to examples of a hanging microfluidic network for spherical microtissue production and culturing, and to the applicability of embodiments using liver and cancer cell lines, but is not restricted thereto.

Further, in the following description the terms "hanging network plate", "network plate", "plate" and "chip" are used synonymously. In addition, the terms "hanging drop compartment", "drop compartment" and "compartment" are used interchangeably. Moreover, in embodiments, the network channel may also be referred to as channel. The term "hanging liquid network" is also referred to herein as "hanging network" or "liquid network".

In one embodiment of the invention, a hanging network plate is provided, including a network surface including at least two hanging drop compartments and at least one network channel fluidly connecting the hanging drop compartments, wherein the at least two hanging drop compartments and the at least one network channel are surface patterns confined by at least one retention structure.

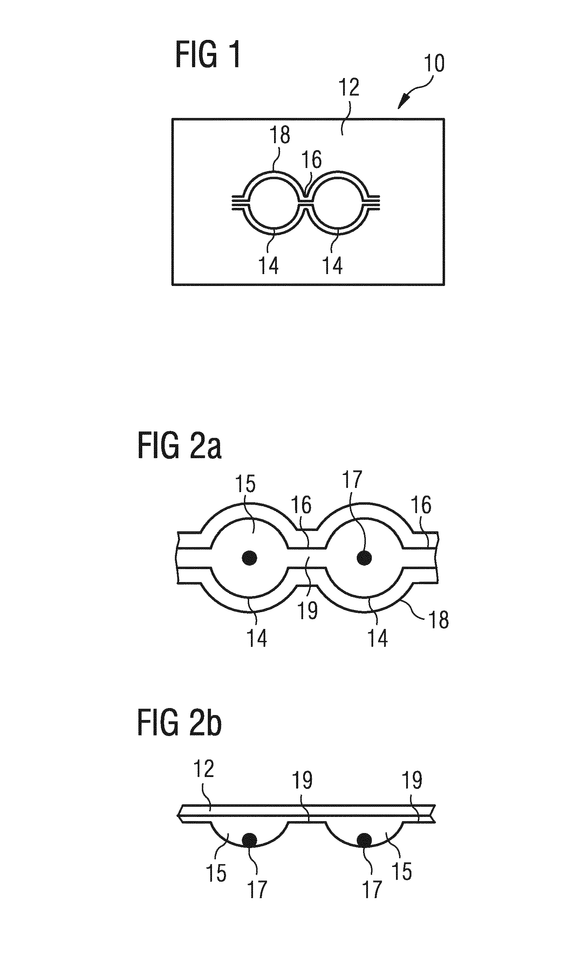

Fig. 1 schematically illustrates a plan view of a hanging network plate 10 according to an embodiment of the invention. Fig. 1 shows the network surface 12 including two hanging drop compartments 14 and a network channel 16 fluidly connecting the compartments 14. The compartments 14 and the channel 16 are surface patterns confined by a retention structure 18.

According to embodiments, the hanging drop compartments and/or the network channels may be grooves provided in the network surface. In further embodiments, the plan view shape of at least one of the hanging drop compartments is circular. At least two of the hanging drop compartments may have different cross sectional sizes and/or different plan view sizes. In some examples, one or more of the hanging drop compartments are not circular at their base to form a spherical drop, but are differently shaped, e.g. longish, to create elongated "sausage"-like compartments. Moreover, at least two or each of the network channels can have the same cross sectional size.

In the embodiment shown in Fig. 1, the compartments 14 are formed as circular grooves and the channel 16 is formed as a linear groove provided in the network surface 12. However, other geometric shapes of the compartments 14 and of the channel 16 are within the concept of the invention. For instance, the compartments may be oval grooves and the channel can be formed as a non-linear, e.g. angled, branched or meandering, groove. In addition, the compartments and the channel may also be formed just by areas of the flat network surface, the areas encompassed by the retention structure. According to embodiments of the invention, the liquid is kept between the retention structures and does not overflow due to capillary and surface forces.

As shown in Figs. 2a (partial bottom plan view) and 2b (partial side view), by arranging the hanging network plate 10 such that the network surface 12 is positioned, in particular unobstructedly positioned, at the bottom of the hanging network plate and introducing at least one liquid into the compartments and/or channels, surprisingly a hanging liquid network formed of liquid drops 15 hanging at the compartments 14 and/or of liquid conduits 19 hanging at the channels 16 is obtained. The step of introducing the liquid may be performed before and/or after the step of arranging the hanging network plate.

In embodiments of the invention, the liquid may for instance include a suspension of cells, tissue and/or proteins. Further, the liquid may contain water, colored water, water-based solutions, cell-culture media, variations of cell-culture media, cell-culture media with added compounds of interest, staining solution for cell staining. Moreover, the liquid may contain any matter of interest. Further, the liquid may contain an oil or an oil-based solution and/or may be an oil based liquid. The cells, tissue and/or proteins may finally sediment to the bottom of the liquid drops 15 to form aggregates 17.

Typically, there are several steps of building and/or maintaining the hanging network: At first, in a loading procedure, a cell suspension (cells plus media) is added to build up the drop, then the cells sediment and form the tissue after a given time. After that, liquid may be added and/or removed through the liquid network. For example, for cell perfusion, to expose cells to different media, defined drugs or other compounds in a defined flow protocol are added. Moreover, cells can also be added after the liquid network has been built up.

As a result, the hanging liquid network including the drops 15 and the liquid conduits 19 is hanging at and is confined to the hanging drop compartments 14 and the channels 16 of the hanging network plate 10. This holding of the hanging network may due to a combined action of one or more of the following effects: The compartments 14 and channel 16 may provide adhesion and/or capillary forces counteracting against gravity and the hydrostatic force in the hanging liquid network. An eventually present selective hydrophilic coating or a hydrophilic material of the compartments and of the channel supports the adhesion of the liquid volume of the drops 15 and of the liquid conduits 19 and works against the hydrostatic force and the gravity. The retention structure 18, for instance a rim or a groove, stabilizes the liquid volume and at least partially confines and defines the liquid volume. Thereby, spreading of the liquid over the whole platform surface is avoided and the liquid network layout is defined. In embodiments, the surface tension allows the liquid to protrude the retention structures at the compartment areas and form stable drops and liquid conduits creating a self-stabilized liquid network. A selective hydrophobic coating may be applied to the area surrounding the compartments 14 and channels 16 of the hanging network plate 10 and may additionally stabilize the liquid volume of the drops 15 and of the liquid conduits 19.

Embodiments of the invention surprisingly allow to pass from single, isolated hanging drops towards a fully interconnected hanging network, where hanging drops are acting as small liquid spaces, for instance to form and host microtissues, and are interconnected with hanging liquid conduits. Therefore, not only the effects of the hanging drops are realized, i.e. a liquid-air-interface at the bottom of the liquid space where cells, tissue and/or proteins cannot attach, but also a continuous media and compound exchange via perfusion in a complex network system. The result is a simple but highly versatile testing platform, for instance for testing drugs.

Embodiments of the invention provide an analytical platform concept, based on an inverted, completely open hanging network plate, in particular a microfluidic system. The hanging network plate exhibits very large design flexibility, is simple with respect to fabrication and operation, and inherently allows for gas-exchange and obviates bubble formation. Moreover, problems with cell adhesion and analyte adsorption, which are critical in biological long-term experiments, are overcome. For cell and tissue cultures, embodiments of the invention provide several advantages. Due to the hanging drop technique cell adhesion on surfaces by the functional use of the air-liquid interface as "well-bottom" is minimized. The completely open system of embodiments of the invention allows for optimal gas exchange during incubation (e.g. oxygen and $CO_2$) and obviates bubble formation in long-term perfusion systems. The liquid phase is accessible at every point in the network for sampling or harvesting of analytes or markers, cells and tissue structures. Cells and tissues can be imaged with high-resolution microscopy from the bottom.

In addition, the invention provides high design flexibility for the creation of complex, interconnected hanging drop networks, and allows for continuous perfusion and media exchange, specific drug dosage, while preserving metabolic inter-tissue communication. This is especially interesting for multi-tissue experiments, where the impact of compounds on several organs is investigated (Body-on-a-Chip) (Esch, M. B., King, T. L. & Shuler, M. L. The Role of Body-on-a-Chip Devices in Drug and Toxicity Studies. Annual review of biomedical engineering 13, 55-72 (2011); Huh, D., Hamilton, G. a. & Ingber, D. E. From 3D cell culture to organs-on-chips. Trends in cell biology 21, 745-54 (2011)).

Further, scaling down using embodiments of the invention allows that surfaces and related forces are getting dominant in relation to volumes: This can be used by relying, for example, on capillary forces and effects of surface tension. Surface interactions can also lead to substantial compound adsorption at the plate walls. However, since most of the surface of the hanging network plate consists of the liquid-air interface, these effects are strongly reduced. No laborious surface coating procedures are required.

In some embodiments, the hanging network plate, the at least one network channel and/or the hanging drop compartments are microfluidic. Also, the hanging drop compartments and/or the at least one network channel may form at least one micropattern. Further, the at least one retention structure may be a capillary retention structure, i.e. may have surface properties and/or dimensions resulting in capillary action on liquids contacting the retention structure. Further, the compartments and/or the channels and/or the retention structure can be formed by micropatterns. Thereby, using the hanging drop method, the hanging network plate of the invention allows (a) parallel production of MT with a single pipetting step, (b) continuous nutrient perfusion and long-term culturing of microtissues and (c) continuous metabolite and compound exchange between different MT of eventually different cell-types for more complex multi-tissue drug studies in a complex microfluidic system.

Hence, the hanging network of embodiments of the invention may be realized by micropatterns on the surface of the inverted hanging network plate, in order to guide the liquid and create an open system of microchannels (Figs. 2a and 2b). The capillary retention structures define the wetted areas and prevent the liquid from spreading over the whole surface. The width of the drops and liquid conduits corresponds to the lateral distance of the retention structures. When liquid is applied, it is drawn through the network by capillary forces. With increasing pressure, drops are formed below the hanging drop compartments, the size of the drops being defined by the surface tension at the liquid-air interface. Thereby, drops and so called microchannels, i.e. liquid microconduits, are formed between the network surface on top and the curved liquid-air interface on the bottom. The height of the drops and of the microconduit arises from the equilibrium between the applied pressure and the surface tension of the liquid. By variation of the lateral expansion of the liquid on the network surface it is therefore possible to define liquid spaces (drops) of different sizes and interconnecting liquid conduits of different cross-sections and, thereby, a complete hanging microfluidic network. The liquid structure is defined by the network surface at the top, the retention structures on the sides and the curved liquid-air interface at the bottom.

Using the hanging network plate of embodiments of the invention, a liquid network of liquid conduits and liquid spaces can be generated on the bottom of the inverted hanging network plate with the help of micropatterns. These micropatterns restrict the liquid from spreading over the whole surface and guide the liquid through a completely open system of microchannels. A microfluidic network thus "hangs" below the patterned plate, whereas the bottom of the liquid conduits and liquid spaces is defined by the surface tension of the liquid-air interface. Such a microfluidic system is very useful e.g. in conjunction with spherical microtissues. Spherical microtissues (MT) are small 3D cell aggregates of 300-500 micrometers in diameter and in recent years became of increased interest for efficacy and toxicity studies in the drug development process of pharmaceutical and cosmetic industry. Cells seeded in a hanging drop sediment to the liquid-air interface and aggregate into a MT.

The retention structure 18, which is schematically indicated in Figs. 1 and 2a, may include at least one element chosen from: an internal lateral edge 22 of the hanging drop compartments and/or of the network channel(s); a hydrophobic rim; a rim surrounding the hanging drop compartments and/or the network channel(s); a rim protruding from an internal lateral edge of the hanging drop compartments and/or of the network channel(s); a retention groove surrounding the hanging drop compartments and/or the network channel(s); a retention groove adjacent and/or surrounding the lateral edge and/or the rim; a capillary stop; and any combination thereof. The retention structure 18 provides, for instance, a change of height with a sharp edge which acts as spreading stop due to capillary force, hence a capillary stop. As a result, the liquid is hindered to flow over the sharp edge.

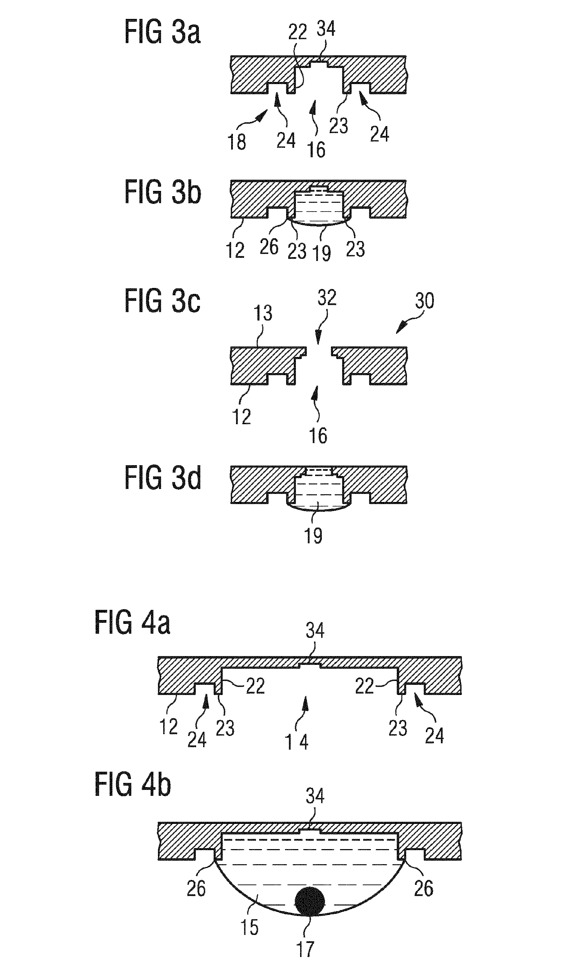

Figs. 3a and 3b schematically show a cross sectional view of the network channel 16 according to embodiments of the invention. Fig. 3a illustrates the channel 16 without an applied liquid, whereas Fig. 3b presents the same channel to which a hanging liquid conduit 19 is attached. The internal lateral edges 22 of the network channel 16 form a retention structure 18 for confining the liquid conduit 19 to the channel 16. As an additional retention structure, two rims 23 protrude from the lateral edge 22 for defining the width of the liquid conduit 19. A further retention structure is formed by two grooves 24 provided in parallel to the channel 16, one groove 24 at each side of the channel 16. In addition, the edges between the rims 23 and the grooves 24 each define a capillary stop 26 avoiding lateral spreading of the liquid, which promotes confinement of the liquid conduit to the channel 16. The combination of the lateral edges 22, the rims 23, the groves 24 and the capillary stops 26 provide a retention structure for confining the liquid conduit 19 to the channel 16, preventing spreading of the liquid provided in the conduit over the network surface. Thereby, the liquid conduit 19 is restricted to the area of the channels 16, preventing wetting of the surrounding network surface by the liquid contained in the liquid conduit 19. However, in embodiments of the invention each feature of the lateral edge 22, the rim 23, the groove 24 and the capillary stop 26 may serve alone as a retention structure.

Figs. 4a and 4b schematically illustrate a cross sectional view of the hanging drop compartment 14 of embodiments of the invention. Fig. 4a shows the compartment 14 without an applied liquid, whereas Fig. 4b presents the same compartment 14 to which a hanging liquid drop including an agglomerate 17 of e.g. microtissue is attached. The compartment 14 is provided with the same retention structures 22, 23, 24, and 26 as the channel 16 shown in Figs. 3a and 3b. Thereby, the drop 15 is restricted to the area of the hanging drop compartment 14, preventing wetting of the surrounding network surface by the liquid contained in the drop 15.

In some embodiments of the hanging network plate, the at least one network channel and/or the hanging drop compartments include at least one guiding channel. Further embodiments are directed to a hanging network plate, in which at least one guiding channel is formed by one or more surface patterns and/or one or more grooves provided in the surface of the at least one network channel and/or of the hanging drop compartments. At least two or each of the guiding channels may have the same cross sectional size. Further, the at least one guiding channel may be provided at the center of the network channels and/or of the hanging drop compartments. At least two guiding channels may be provided and fluidly interconnected to each other. Moreover, at least one of the hanging drop compartments may have two or more fluidly interconnected guiding channels being positioned at an angle to each other.

Figs. 3a to 3b and 4a to 4b show cross sectional views of embodiments including guiding channels 34 centrally provided as linear grooves in the surface of the at least one network channel and/or of the hanging drop compartments.

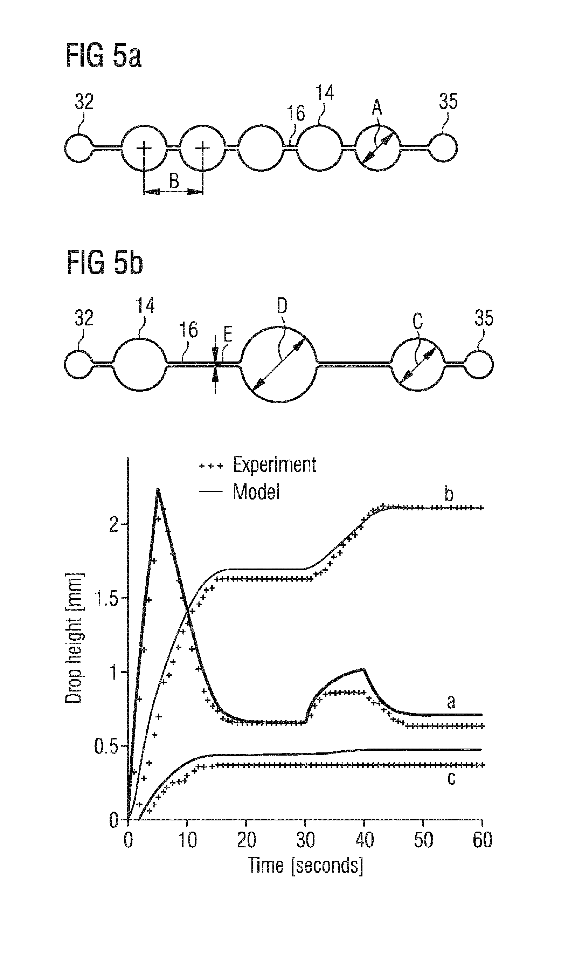

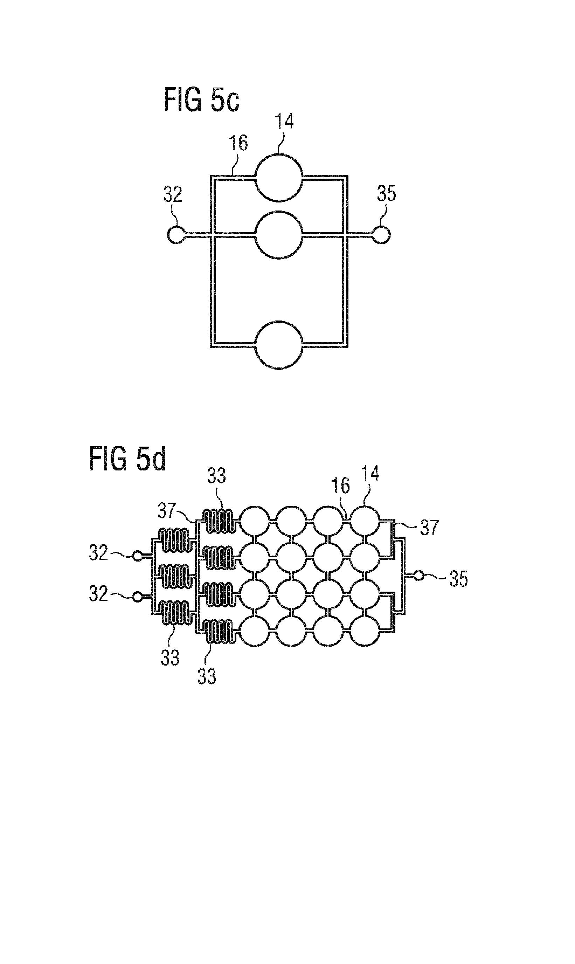

In the hanging network plate of embodiments, the hanging drop compartments may be arranged in at least one array. Examples of such arrays are schematically illustrated in Figs. 5a to 5d, the retention structures not shown. In Figs. 5a and 5b, a plurality of hanging drop compartments 14 are arranged in linear arrays. The compartments 14 are interconnected serially by a channel 16, one channel 16 between two compartments 14. The array of Fig. 5a includes five compartments 14 of identical size, whereas the array of Fig. 5b includes three compartments 14 of two different sizes. Fig. 5c shows an array of three hanging drop compartments 14 interconnected by channels 16 by means of a parallel arrangement of the compartments 14. Fig. 5d depicts a 4x4 array of compartments 14 interconnected serially and in parallel by channels 16, at least one channel 16 between two neighboring compartments 14.

According to one embodiment of the hanging network plate, the hanging network plate includes at least one inlet for liquids, the inlet being fluidly connected to at least one of the hanging drop compartments and/or to at least one of the network channels. At least one of the inlets for liquids may be provided in another surface of the hanging network plate. Further, at least one of the inlets for liquids can be provided in another surface of the hanging network plate, the other surface being provided diametrically opposed to the network surface.

For instance, in Figs. 3c and 3d an example of a hanging network plate 30 is shown, including the network surface 12, a back surface 13 diametrically opposed to the network surface 12, a channel 16 provided in the network surface 12, and an inlet 32 provided in the back surface 13. Fig. 3c illustrates the channel 16 without an applied liquid, whereas Fig. 3d presents the same channel 16 to which a hanging liquid conduit is attached. The inlet 32 is fluidly connected to the channel 16. By introducing liquid through the inlet 32, the channel 16 is filled to form a liquid conduit 19.

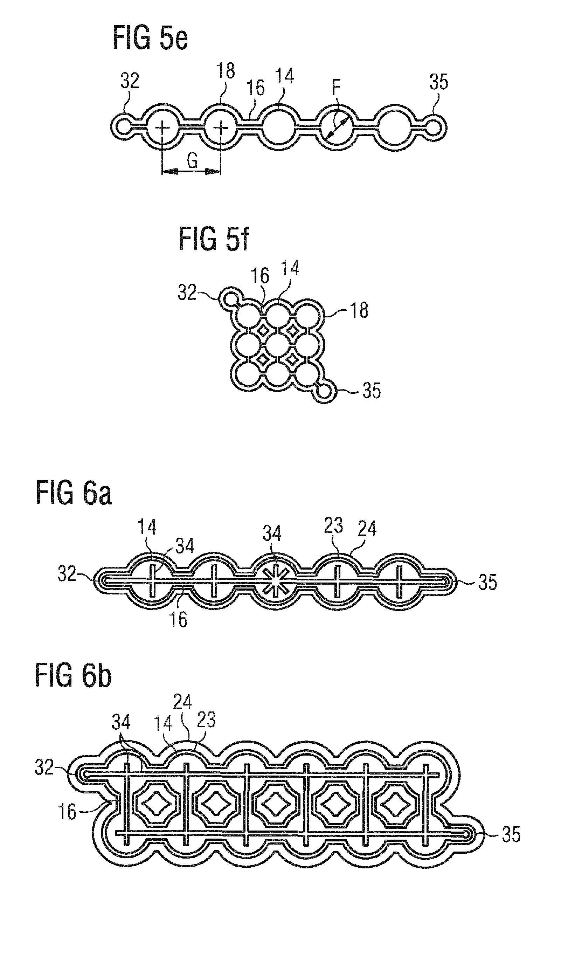

Figs. 6a and 6b illustrate arrays of hanging drop compartments 14 interconnected by channels 16, the arrays having a linear configuration (Fig. 6a) and a 5x2 configuration. It is shown, that the hanging drop compartments 14 may have two or more interconnected guiding channels 34 being positioned at an angle to each other. Further, the inlets 32 and outlets 35 of the arrays may also include guiding channels interconnected to other guiding channels of the array. The guiding channels help the liquid to quickly distribute in large hanging networks.

In some embodiments of the hanging network plate the hanging drop compartments are circular and have a diameter of about 2 to 7 mm, preferably 3 to 6 mm, more preferably 3.5 to 5 mm. Further, the centers of neighboring hanging drop compartments may have a distance of about 2.5 to 30 mm, preferably 3 to 10 mm, more preferably 4.5 to 9 mm, from each other. However, this distance can vary and may be larger than 9 mm. Moreover, the hanging drop compartments can have a depth of about 0.1 to 2 mm, preferably 0.2 to 1.5 mm. In further embodiments, the network channels may have a width of about 0.1 to 1.0 mm, preferably 0.1 to 0.4 mm, more preferably 0.2 mm; and/or a length of about 0.05 to 5 cm, preferably 0.2 to 4 cm, or more than 5 cm. However, the length of the network channels can vary depending on the desired network (see Fig. 5c). Further, the network channels may have a depth of about 0.1 to 2 mm, preferably 0.2 to 1.8 cm. In further embodiments, the guiding channels may have a width of about 0.1 to 1 mm, preferably 0.2 to 0.8 mm. Moreover, the guiding channels may have a depth of about 0.1 to 0.5 mm, preferably 0.2 to 0.4 mm. In addition, the depths of the hanging drop compartment and of the network channels can be different. Further, the cross sectional shapes of the network channels may be square, but can have rounded corners or may be half-circular, depending on the fabrication process.

The hanging network plate of some embodiments may be rigid. Further, the surface patterns and/or retention structures may be of a soft material. Moreover, the hanging network plate may include a material chosen from polymer and/or metal. For instance, a silicone such as poly(dimethylsiloxane) (PDMS) may be used. In particular, PDMS is used as rapid prototyping material. However, any material, for instance plastics, metals, silicones, epoxies, etc., that can be casted from a mold, injection molded, or conventionally machined with the specified dimension may be used. In particular, the hanging network plate may be produced by casting polymer materials from a negative mold, injection molding and/or structuring by a (hot) embossing process and/or by structuring polymers and/or metals using machining, milling, shape cutting, and/or chipping technology.

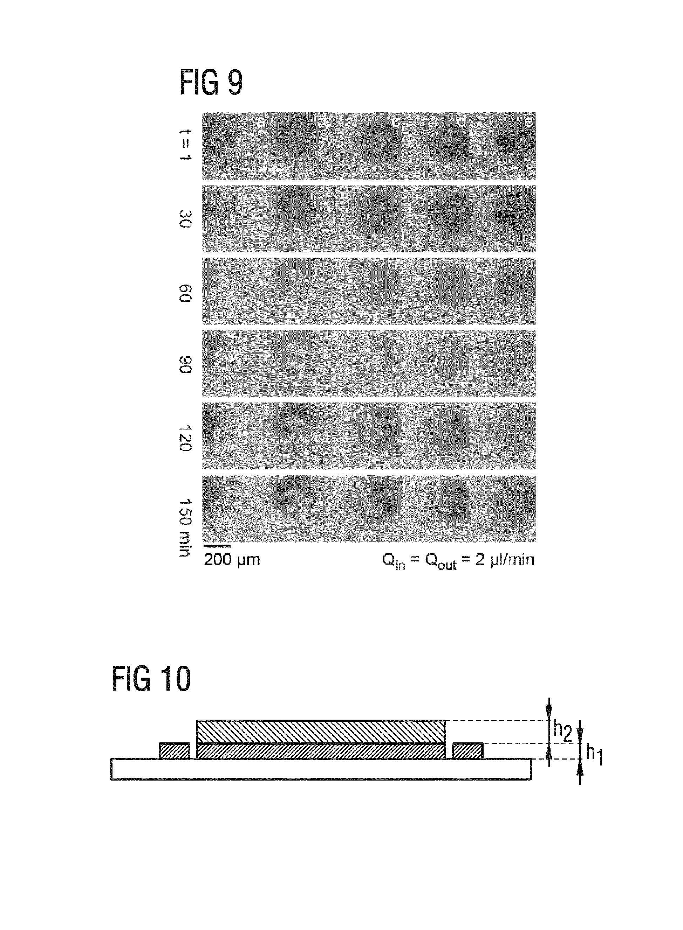

According to another embodiment, a method for producing a hanging network plate of any of the preceding embodiments is provided, including forming a hanging network plate having a network surface including at least two hanging drop compartments and at least one network channel fluidly connecting the hanging drop compartments, wherein the at least two hanging drop compartments and the at least one network channel are surface patterns confined by at least one retention structure; and/or forming a negative mold of the hanging network plate and casting the material of the hanging network plate in the mold. An example of the negative mold is shown in Fig. 10, illustrating the area of the negative mold that forms the hanging drop compartment 14 shown in Fig. 4, however without a guiding channel. For instance, a rapid prototyping method may be used for forming the negative mold. Further, the simplicity of the method allows a variety of other production techniques such as hot embossing or injection molding.

In the method of the preceding embodiment, the negative mold may be silanized or vapor silanized before casting. Thereby, adhesion to the negative mold is reduced. Further, holes may be provided, e.g. punched, into the plate at the inlet positions. The casted plate may be cleaned, in particular selectively cleaned, and/or treated, in particular selectively treated, with oxygen plasma. This promotes wettability of at least parts of the plate. At least parts of the network surface and/or the retention structures of the plate may be covered with a thin layer during the oxygen plasma treatment, except for the hanging drop compartments (14) and the at least one network channel (16). Thereby, the retention structure may be made and/or kept hydrophobic, preventing liquid overflow. In some examples, the retention structure may be made or kept hydrophobic while the hanging drop compartments and channels are made or kept hydrophilic. Alternatively, the retention structure may be made or kept hydrophilic while the hanging drop compartments and channels are made or kept hydrophobic, which may be useful for oil-based liquids or solutions.

Further embodiments are directed to a use of the hanging network plate of any of the preceding claims for producing and/or culturing cells or tissue, for engineering tissue, for forming spherical microtissue and/or cell aggregates, for performing assays, for performing in-vitro cell-based assays, for performing toxicity assays, for performing protein crystallization, and/or for forming material aggregates. Due to use of the plate of embodiments, it is possible to apply continuous flow, programmed flow-protocols, directed and guided flow through the different compartments in any order. Hence, the use of embodiments allows a fluid communication of the compartments with each other.

According to embodiments, a method for producing a hanging liquid network including at least one liquid drop and/or a liquid network, includes arranging the hanging network plate of any of embodiments of the invention such that the network surface is positioned at the bottom of the hanging network plate. Thereby, the network surface may be positioned unobstructedly and/or freely at the bottom of the hanging network plate.

In a further embodiment, at least one liquid is introduced into at least one of the inlets of the hanging network plate, and/or at least one liquid is removed. For instance, the liquid may be removed from at least one outlet of the hanging network plate or directly from the liquid network, drops or liquid conduits. The inlet may also be used as an outlet. At least one of the liquids may include a suspension of cells, tissue and/or proteins and/or another compound interfering with the cells, tissue and/or proteins located in the liquid drop and/or liquid network. Thereby, a hanging network may be produced, once the channel and the drop compartments are completely filled, and drops may be formed below the drop compartments and continuously grow while liquid is added. The hanging drop network can also be built up without cells for other applications.

Embodiments of the method for producing a hanging liquid network may include perfusing the hanging liquid network with liquid, in particular for maintaining a culturing condition; and/or exposing matter in the at least one liquid drop to a compound; and/or removing liquid from the liquid drop and/or liquid network for analysis. For example, the hanging network plate of the invention allows (a) parallel production of microtissues (MT) with a single pipetting step, (b) continuous nutrient perfusion and long-term culturing of microtissues and (c) continuous metabolite and compound exchange between different MT of eventual different cell-type for more complex multi-tissue drug studies.

Examples

Experimental Section

*Chip fabrication and operation:* For producing examples of hanging network plates according to the invention, microfluidic structures have been created through casting poly(dimethylsiloxane) (PDMS, Sylgard 184, Dow Corning, USA) using a microfabricated SU-8 mold shown in Fig. 10. SU-8 is a negative, epoxy-type, near-UV photoresist based on EPON SU-8 epoxy resin (from Shell Chemical). The mold itself has been fabricated with two layers of SU-8 spin-coated consecutively onto a 4-inch silicon wafer to achieve the desired overall thickness ($h_1$ = 200 $\mu$m, $h_2$ = 250 $\mu$m) and UV-exposed through a transparency mask to initiate cross-linking. The transparency mask includes the layout of the microfluidic network as transparent features, the rest is opaque. During UV-exposure these structures are transferred into the spin-coated SU-8, which cross-links only when exposed to UV. Unexposed SU-8 is washed away during the preceding development step. The obtained SU-8 master constituted the negative of the final microfluidic structure in the PDMS. Before PDMS casting the molds have been coated with trichloro (1*H*, 1*H*, 2*H*, 2*H*-perfluorooctyl)silane (Sigma-Aldrich, Switzerland) in a vapor silanization process in order to reduce adhesion. Cured PDMS was cut to desired sizes, and fluidic access holes were punched at the inlet and outlet sites. For better stability and planarity, the PDMS layer was bonded to a microscopy slide featuring drilled liquid access holes after oxygen plasma activation.

Prior to the experiments, the microfluidic chips were cleaned with soap, rinsed with acetone and isopropanol and dried using an air gun. Wettability of PDMS was increased by applying oxygen plasma. In order to ensure drop stability, the patterned surface was covered with a thin PDMS layer featuring openings only at the drop sites. This procedure resulted in a localized surface modification that keeps the rim around the drop site hydrophobic and prevents liquid overflow.

*Operation and setup:* All experiments have been performed on a custom-made chip holder, placed in a covered 1-well culture dish. Perfusion flow-rates were controlled using a precision syringe pump connected through commercial PTFE tubing (ID 0.5 mm, OD 1.0 mm).

*Microscopy:* Time-lapse experiments have been performed on a NIKON Ti Eclipse inverted fluorescence microscope with a motorized xy-stage, and a stage-top incubator to control temperature (37 °C), humidity (100 %) and $CO_2$ levels (5%). The chips were placed on a custom-made chip holder in a covered 1-well culture dish together with a PBS-soaked cotton pad. This way, drop evaporation could be reduced to 70 nl/h per drop (see Table 1). Bright-field and fluorescence time-lapse images were acquired with a Hamamatsu ORCA Flash 4.0 camera every ten minutes using automated stage positioning and software-based autofocusing.

*Cell culture:* Human hepatic carcinoma (HepG2) cells and human colorectal carcinoma (HCT-116 eGFP) were cultured in RPMI 1640 growth medium (PAA, Austria), supplemented with 10% fetal bovine serum (FBS, Sigma-Aldrich) and 100 $\mu$g/ml penicillin and 10 $\mu$g/ml streptomycin. The culture was maintained in a humidified incubator at 37 °C in 5% $CO_2$ (Heracell 150i, Thermo Scientific, USA).

Cell culturing was carried out according to routine protocols. Cells were sub-cultured until reaching 80% confluence. Before starting an experiment, the cells were cultured to confluence, and then digested with 0.025% trypsin/EDTA solution (in growth medium). The cell suspension was centrifuged, and the cell pellet was washed twice with PBS. The cells were re-suspended in fresh growth medium. Cell density was measured using a hemocytometer and adjusted according to the requirements of the experiment.

*Microtissue fabrication:* A suitable medium volume of defined cell density (35'000-40'000 cells/ml) was pipetted by hand directly into the chip via one of the liquid access holes of the inlet(s) while applying the optimal flow-rate as stated in Table 1. No perfusion was applied.

*Live/dead staining:* Perfusion staining was performed using the following flow protocol after having connected all tubings and the syringe pumps at the inlet and outlet. Flow-rates were kept constant at 1.5 $\mu$l/min or -1.5 $\mu$l/min. 70 $\mu$l of serum-free medium was first introduced to reduce background fluorescence. Then 40 $\mu$l of staining solution were applied continuously, followed by a washing step with 140 $\mu$l of phosphate-buffered saline (PBS). The live/dead staining solution consisted of 1.33 $\mu$l Calcein AM (Life technologies), 10 $\mu$l of propidium iodide (Sigma), stock solution (4 mM), diluted in serum-free RPMI 1640 media (+ 1% Penicillin / Streptomycin).

**Working Examples**

Using examples of the embodiments shown in Figs. 5a to 5f and formed by the above mentioned *Chip fabrication,* experiments, utilizing de-ionized water as a liquid, were performed on the respective inverted hanging network plate for evaluation of the drop volume and radius, the loading rate of the liquid, the perfusion rate in the drops, the evaporation rate from the drops and/or the distribution of suspended beads. The height of the drop was measured using an inverted microscope (NIKON Ti Eclipse). The variation of the drop height was extracted from the z-position of the objective when focused on the lowest point of the drop or from images acquired from the side using a commercial digital camera. The size of the microtissue was measured optically using a microscope (NIKON Ti Eclipse). Radii were calculated using the measured dimension in z direction (z = vertical). Added volumes were measured using a precision syringe pump (Harvard Apparatus). The evaporation-rate was calculated using the height variation of the drop over time. Bead distributions were counted manually from microscopy pictures.

In each of Figs. 5a to 5f, the layouts of hanging drop compartments 14 and at least one network channel 16 including at least one retention structure 18 according to embodiments are schematically illustrated. Figs. 5a to 5d are illustrated without showing the retention structure 18. In Figs. 5e and 5f the retention structure 18 is schematically indicated.

Figures 5a and 5e schematically show two simple layouts of 5 drops, aligned in serial configuration applying a typical 96-well-plate pitch and 384-well-plate pitch, respectively, and interconnected with liquid conduits attached to 200μm wide, open channels. Drops are formed below the circular compartments of the inverted plate, the drop size being defined by the base diameter of the compartments. A retention structure 18 formed by a 150μm wide rim (not shown in Fig. 5a) prevents the liquid from overflowing. At the inlet 32, the channels and drop compartments were accessible from the top with a pipette or via tubes. Relatively narrow liquid conduits attached to the channels 16 were interconnecting the wider drops. The drop was defined by a cylindrical, recessed part, and protrudes from the hanging network plate. Further layouts included a parallel drop configuration (Fig. 5c), and two array formats (Figs. 5d and 5f). The drop array formed using the example of Fig. 5f consisted of 9 drops at 4.5 mm pitch, whereas the array formed by using the example of Fig. 5d consisted of 16 drops at 4.5 mm pitch, combined with a gradient generator for parallel drug compound testing. The examples of Figs. 5a to 5f have been casted in PDMS using the SU-8 mold described above, each including a retention structure 18 formed by a 150μm wide rim (not shown in Figs. 5a to 5d).

Using the array of Fig. 5a, a microfluidic hanging network including connected hanging drops was tested. Experiments have been done by using a network plate according to the invention having a serial configuration of five drop compartments with a base diameter A of 3.5 mm and a pitch B of 4.5 mm. A 1mm long and 200μm wide channel 16 connected one drop compartment to the next. The system was initially filled with a liquid consisting of colored water for better visibility using a low inlet flow-rate of 10 μl/min. Once the grooves of the channel and drop compartments were completely filled, drops were formed below the circular drop compartments and continuously grew while liquid was added. The drop radius was decreasing until it reached the minimal radius, which corresponds to the radius of the drop base. At this stage the drop had the shape of a semi-sphere, if deformation by gravity is neglected. Further addition of liquid resulted in an increase of the drop radius. The pressure in the drop with respect to ambient pressure is defined by the Young-Laplace equation:

$$\Delta p = \frac{2\gamma}{r}$$

with $\gamma$ being the surface energy per area. $\Delta p$ is inversely proportional to the drop radius r. As a consequence the pressure in all connected drops is automatically equilibrated during filling of the microfluidic hanging network, because every drop "tries" to minimize its surface energy and "pushes" liquid to the next drop, if the radius of the next drop is larger and its volume is smaller, respectively. This results in equal drop volumes after chip filling, even if only a single inlet is used for liquid supply.

Continuous liquid exchange can be performed by applying equal flow-rates at inlet 32 and outlet 35. An externally applied flow induces a pressure drop in the microfluidic system according to the flow resistances of the connecting liquid conduits.

Using the embodiment shown in the top illustration of Fig. 5b, in a hanging network three drops of two different sizes with a base diameter of 3.5 and 5 mm were connected in series. The two outer hanging drop compartments 14 have a diameter C of 3.5 mm, whereas the central drop compartment 14 has a diameter D of 5 mm. The channel 16 interconnecting the compartments 14 has a width E of 0.2 mm and a length between the compartments of 4.5mm. The bottom illustration of Fig. 5b shows a diagram presenting the drop heights as a function of time during formation of the hanging network after feeding the colored water into inlet 32. Close-up views of the drops produced in the hanging network showed almost circular shapes, affirming that gravity plays a minimal role as compared to surface tension (not shown here). An initial 5-second liquid flow pulse of 300 μl/min leads to a sharp increase in the height of the first drop (curve a), because the resistance of the liquid conduit towards the second drop (curve b) was too high and the pressure difference between the first and the second drop was too low to move the liquid forward at a similarly high flow-rate. After 20 seconds the system was equilibrated and at steady state. The different heights of the first drop and the third drop (curve c), even though they had same base diameter, resulted from the 20 μl/min perfusion. Application of a second pulse of 60 μl/min yielded a similar behavior. This shows that the first drop acts as a buffer and dampens out sharp flow changes in analogy to a low-pass filter. The experimental results of the perfusion test can be seen in the bottom illustration of Fig. 5b and correlated with a corresponding model calculation which is not described herein.

The array embodiment schematically illustrated in Fig. 5c provides a parallel drop configuration. While media or test compounds in serial drop configurations flow from one drop to the next and allow for studying inter-tissue reactions, this may not be needed for other biological questions. In the example of Fig. 5c, three drops attached to hanging drop compartments 14 of a base diameter of 3.5mm may be perfused in parallel with liquid conduits attached to the channels 16 of different lengths of 3, 7 and 11 mm, resulting in different flow resistances. All widths of the channels 16 are 200 $\mu$m. This configuration provides continuous perfusion without any interference between the drops. Further, liquid conduit resistances to and from a certain drop can be chosen in a way to adjust the flow-rate in this specific drop without altering the drop size. For instance, an initial drop volume of 8 $\mu$l stayed constant in all 3 drops at an applied perfusion rate of 10 $\mu$l/min. The flow rates, however, were different in all drops. Increasing the flow rate at the inlet to 20 $\mu$l/min resulted in a change of the flows in all 6 branches. The difference between inflow and outflow in each drop was the same and resulted in the same liquid volume increase in all three drops. Switching the inflow back to 10 $\mu$l/min reset all branch flow-rates, preserving the larger but still identical drop volumes in all three branches. Such a simple system for example allows studying the effect of media exchange on microtissue viability or controlled adjustment of drop size of the microtissues.

The array example schematically shown in Fig. 5d includes a gradient generator, in order to produce a gradient array for drug exposure. This example includes a 4x4 array of hanging drop compartments 14 with a base diameter A of 3.5 mm and a pitch B of 4.5 mm and interconnected by channels 16 of a width of 0.2 mm and a length of 1 mm in parallel and serially and in addition by further branches 37 partially including meandering regions 33 of the channels 16. The regions 33 form the gradient generator (Jeon, N. L. et al. Generation of Solution and Surface Gradients Using Microfluidic Systems. Langmuir 16, 8311-8316 (2000)). The gradient generator allows the production of four different concentrations of a target compound of 0, 33, 67 and 100% of the original concentration by making use of integrated flow splitting and mixing structures. The four concentrations were applied in parallel to the four initial drops using a flow rate of 1 $\mu$l/min. The flow-rates in all drops were equal. All micropatterns including the meandering network channels were open to the bottom. This system therefore allows, for example, the exposure of 16 hanging drops, produced in parallel on the hanging network plate, to four different drug concentrations. The system features stable drop formation and is compatible with microfluidic approaches relying on laminar flow characteristics.

As further examples, a linear array of liquid drops as shown in Fig. 5e and a 3x3 array of liquid drops as shown in Fig. 5f are formed using corresponding network plates. In the following, the example according to Fig. 5a is called S2, the example according to Fig. 5e is called S1, and the example according to Fig. 5f is called A1. In the array of Fig. 5e, five drop compartments 14 had each a base diameter F of 5 mm and a pitch G of 9 mm, and a 4 mm long and 200 $\mu$m wide channel 16 connected one drop compartment to the next. In the array of Fig. 5f, nine drop compartments 14 with each a base diameter of 3.5 mm and a pitch H of 4.5 mm were connected to each other by a 1mm long and 200$\mu$m wide channel 16.

The layouts S1, S2, and A1, were experimentally characterized with regard to drop volumes, loading behavior, stability and perfusion features. In order to evaluate the distribution of matter suspended in the colored water, polystyrene beads having a diameter of 10 $\mu$m have been suspended therein. The results are summarized in Table 1. Using the production method described above, drop volumes of 28, 14 and 12 $\mu$l with good stability have been achieved. The loading speed during the initial filling strongly depended on the drop size and inter-drop flow resistance. Large drops and high resistances led to longer times for drop-size equilibration. The stability of the drops reached an optimum for S2 and A1 layouts with 200-$\mu$m-wide interconnections.

**Table 1.** Experimental parameters of the basic layouts S1, S2, and A1

| | Drop diameter [mm] | 5.0 | 5.0 | 3.5 | 3.5 | 3.5 |
|---|---|---|---|---|---|---|
| | Chip design | S1 | S1 | S2 | S2 | A1 |
| | | (Fig. 5e) | | (Fig. 5a) | | (Fig. 5f) |
| | Channel (w$\times$l)[mm] | 0.4$\times$4.0/0.2 $\times$ 4.0/0.4$\times$1.0/0.2$\times$1.0/0.2$\times$1.0 | | | | |
| | Total chip volume [$\mu$l] | 140 | 140 | 70 | 70 | 110 |
| | Single drop volume [$\mu$l] | 28 | 28 | 14 | 14 | 12 |
| Loading rate [$\mu$l/min] [a] | | 250 | 50 | 500 | 350 | 400 |
| Loading behavior | | + | - | +++ | +++ | ++ |
| Perfusion rate [$\mu$l/min] [b] | | 1 | | | 1 | |
| Flow rate [$\mu$l/min] [c] | | 30-50 | 40 | 5 | 5 | 5 |
| Drop stability [d] | | ++ | ++ | + | +++ | +++ |
| Evaporation rate [$\mu$l/h] [e] | | 1.1 | 1.1 | 0.35 | 0.35 | N/A |

(continued)

| Bead distribution (9000 beads/ml) | | | | | |
|---|---|---|---|---|---|
| Optimal loading | N/A | N/A | N/A | 4.9% | 5.9% |
| Slow loading | | | | 14.0% [f] | |
| Fast loading | | | | 7.9% [f] | |

[a] Optimal flow-rate experimentally evaluated to achieve best drop homogeneity.
[b] Maximal experimentally tested perfusion flow-rate without visible movement of particles in a drop.
[c] Maximal experimentally tested perfusion flow-rate without dragging particles from a drop to the next.
[d] Stability of the individual droplets when transporting or slightly tilting the chip.
[e] Under experimental conditions on an inverted microscope in a stage top incubator (controlling relative humidity at 100%).
[f] Bead distribution variations for slow and fast loading flow-rates are higher due to accumulation of beads in the first drop.

A drop array version based on the hanging network plate of layout A1 (Fig. 5f), which is a matrix of 3 by 3 water drops, which also show homogenous bead distribution after loading, was extended to a larger format, such as an array of 16 by 12 drops. The latter represents half of a 384-drop-plate, which is commensurable with standard well plate formats. Interconnecting the drops led to equal-size single drops over the whole array even though loaded through only a single or a few inlets. This way, a large number of microtissues can be produced while liquid handling is reduced to a single pipetting step by using, for example, a multi-channel pipette.

**Bead Distribution Test**

Using hanging network plates having the layouts S1 and S2 (Figs. 5b and 5a), a bead distribution in a hanging liquid network of water containing polystyrene beads was tested. The use of embodiments of the invention for the production of spherical microtissues in analogy to the hanging drop method requires controlled, reliable and reproducible distribution of cells in the individual drops, when the cell suspension is initially loaded into the liquid network. Especially in drug discovery, identical-size microtissues are crucial for comparable studies. The dimensions of the microfluidic structures of embodiments are optimized with regard to achieving a uniform distribution of beads and cells in the drops. A well-mixed cell (HCT116 cell line) /bead suspension of specific cell concentration has been loaded in a single pipetting step into the microfluidic hanging network plate S2. Optimal loading rates for the different basic designs are listed in Table 1. The relatively high flow-rates resulted in a rapid filling of the chip. Once loading has been completed, beads or cells sedimented at the bottom of the respective drop, where they tend to agglomerate. Lower flow-rates led to pre-sedimentation during the filling process, whereas higher flow-rates led to excessive increase of the first drop. Both scenarios resulted in higher bead and cell numbers in the first drop.

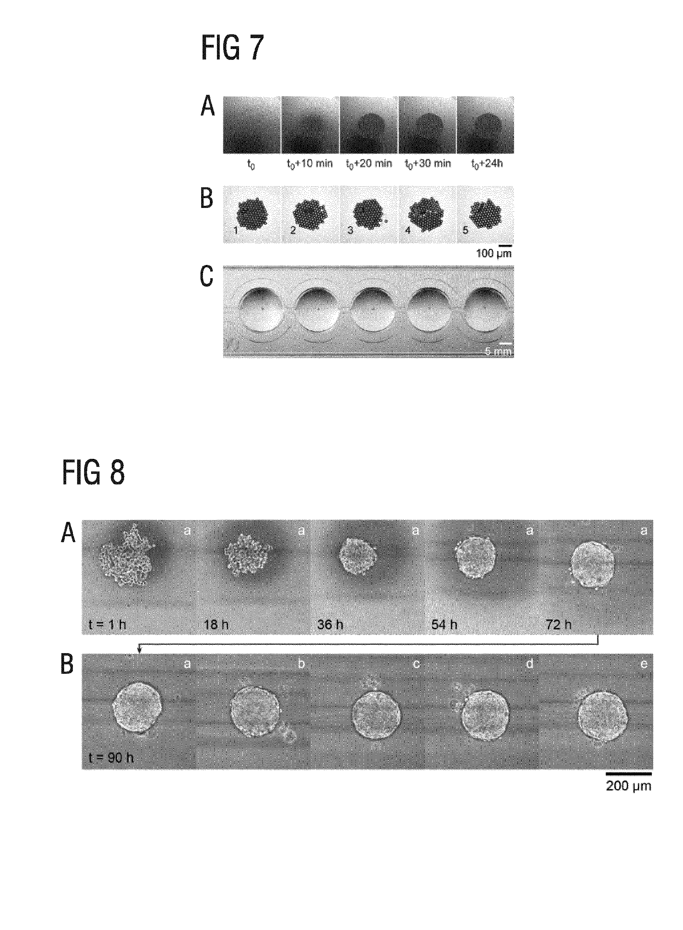

Figs. 7A to 7C show the bead distribution after chip loading. Fig. 7A shows microscope images of the sedimentation of an arbitrary concentration of $10\mu m$ beads in one of the drops at different time points. Fig. 7A illustrates a time-lapse imaging showing sedimentation of an arbitrary concentration of $10\mu m$ beads in 5mm drops (S1 layout). Repeated (n=5) low variations in the number of beads on the order of 5% were observed in the different drops. Figs. 7B and 7C present an example of loading a chip of version S2 with $20\mu m$ beads (9000/ml) while using an optimal flow-rate (see table 1). Fig. 7B shows the homogenous distribution of $20\mu m$ beads (9000/ml) in the S2 layout. Fig. 7C is a top view of the full chip with aggregated beads at the bottom of each drop.

**Parallel Microtissue Formation Test**

Using a hanging network plate having the layout S2 (Fig. 5a), a parallel microtissue formation was tested. Highly homogenous microtissues have been formed using the chip, as illustrated in Fig. 8. After placing the chip on an inverted microscope, a cell suspension of HCT-116 at a concentration of 35'000 cells/ml in RPMI cell culture medium was added and incubated for nearly 4 days. The formation of microtissues has been monitored using automated time-lapse imaging. Figure 8A shows images of the formation and growth of the microtissue in the first drop a at time intervals of 18 hours. Seeding concentration was 490 cells/drop (= 35'000 cells/ml). Microtissue growth after tissue formation at ~48 hours was 9.6 $\mu$m/h for the given initial diameter. Figure 8B shows final images, taken 90 hours after cell loading using a single pipetting step, of all 5 microtissues that have been formed in parallel in drops a to e. The microtissues feature almost identical sizes and a similar, compact morphology. For this specific experiment, nearly equal microtissue diameters of 224.6 ± 3.5 μm were measured, i.e. the final MT size in a stage top incubator was 224.6±3.5 μm.In repeated experiments size variations of about 5 % were achieved.

**Continuous Microtissue Perfusion Test**

The features of controlled perfusion and compound exposure of cells and microtissues in the interconnected drops are demonstrated in Fig.9, which shows an in-chip fluorescent staining through perfusion using a hanging network plate having the layout S2. HepG2 cells have been stained during the aggregation process directly in the drops of cell culture medium by using a continuous-flow protocol, wherein the inlet flow rate $Q_{in}$ and the outlet flow rate were equal to 2 μl/min. During 150 min, a solution containing the life stain CalceinAM and the dead stain propidium iodide has been perfused through the 5 drops, in which the cells were aggregating. The microscope images of Fig. 9 (overlay of bright-field, green fluorescence and red fluorescence pictures) illustrate the passage of the slightly fluorescent staining solution through the 5 drops. The low flow rate of 2 μl/min ensured that cell movement within the drops was minimized and that the cells were not dragged from one drop to the next. After 150 min, a clearly observable staining of predominantly living cells in the drops was apparent.

**Results Summary**

The examples show that embodiments of the invention allow to pass from single, isolated hanging drops towards a fully interconnected hanging network, where hanging drops are acting as small liquid spaces, for instance to form and host microtissues, and are interconnected with hanging liquid conduits. Therefore, not only the effects of the hanging drops are realized, i.e. a liquid-air-interface at the bottom of the liquid space where cells, tissue and/or proteins cannot attach, but also a continuous media and compound exchange via perfusion in a complex network system. The result is a simple but highly versatile testing platform, for instance for testing drugs. The invention allows forming a fully interconnected hanging microfluidic network. In the exemplary application of tissue engineering, the hanging drops are acting as small liquid spaces interconnected with hanging liquid conduits. Using the hanging drop method - a liquid-air-interface at the bottom of the liquid compartment where cells cannot attach - the hanging network plate of the invention additionally allows (a) parallel production of MT with a single pipetting step, (b) continuous nutrient perfusion and long-term culturing of microtissues and (c) continuous metabolite and compound exchange between different MT of eventual different cell-type for more complex multi-tissue drug studies.

Embodiments of the present invention further enable the fabrication of analytical platforms, which do not only allow for reliable production of spherical microtissues in larger arrays with a low number of handling steps, but also enable microfluidic networks that comprise different tissue types. Moreover, the different tissues can be interconnected according to their physiological context and be cultivated under controlled perfusion. The invention not only allows for testing of compounds or candidate drugs on a single cell type, but also for studying the interactions of a specific drug compound with a multi-organ-like system. Embodiments of the invention exhibit high flexibility in design, high accessibility to optics, and the option to perform liquid sampling and cell harvesting. Further, the integration of sensor systems is possible.

The examples provide evidence of the advantages provided by the hanging network plate of the invention, in particular as an analytical platform. Hanging drops for the production of spherical microtissues rely on the liquid-air interface to prevent cell adhesion and enable simple aggregation of cells to 3-dimensional tissue structures. The simplicity of production and handling together with the better mimicking of in-vivo-like cell behavior, as compared to 2D single-layer cell cultures, renders spheroids very attractive as tissue models for the drug discovery process to obtain predictive and reliable data at an early stage. Using embodiments of the invention, a better forecast of organ-specific drug efficacy and toxicity significantly increases the testing efficiency and reduces development cost. Tissue-based methods may even gain more importance in view of ongoing efforts to develop alternatives to animal testing. Linking the isolated hanging drops using embodiments of the invention to obtain an interconnected network, in particular a microfluidic network, entails several benefits: (a) Controlled perfusion is possible and allows for nutrient supply and drug dosage. Further, the connecting channels enable transportation of substances between different drops. (b) Microtissue production can be further automated and simplified, due to a substantial reduction of pipetting steps. (c) Microtissue production and cultivation are combined in the same platform.

The implementation of embodiments of the invention as completely open system of e.g. microfluidic structures extremely simplifies chip production, as only the patterning of a single surface of a green body plate or chip is required. No additional cover for channel closure is needed. This allows for using mass fabrication methods, such as injection molding. Further, the open system is directly accessible for optical imaging and liquid sampling, as well as for microtissue harvesting for further analysis. Gas exchange is inherently ensured and undesired bubble formation is inexistent. The large liquid surface area leads to continuous evaporation, which may affect analyte concentrations in the liquid or entail changes in drop volumes and flow dynamics. These effects are comparable to those in standard hanging-drop platforms, where solutions already exist, mainly by creating a water-saturated, surrounding atmosphere. Moreover, the perfusion option of the system according to embodiments of the invention allows for continuous exchange or even compensation of the evaporation via an increased inflow.

The dynamic characteristics of open, hanging network systems, e.g. microfluidic systems, are different when compared to closed ones. The main reason is that each drop, which represents a liquid compartment in the network, is a dynamic element concerning its volume and internal pressure. This renders the system characteristics less intuitive and more difficult to predict. The specific dynamic characteristics of the hanging drop are given by the surface tension at the liquid-air interface. The acting forces equilibrate all connected drops to the same drop radius, if no external flow is applied, and inherently stabilize the drop network. The retention structure of embodiments of the invention, e.g. a rim, which defines the lateral dimensions of the drops and/or liquid conduits, can be varied with great flexibility in order to create a variety of networks. The drop structures have to be designed in order to provide maximal functionality and, at the same time, high drop stability. Small drop radii, for example, create larger surface forces making them less prone to drip off and increase drop dynamics. However, higher pressures are needed to generate such smaller drops and less media volume is available to the microtissue. Narrow interconnecting channels lead to increased flow resistances between the drops, which limit the flow-rates due to the resulting need of a larger pressure. Narrow channels, however, also render the system more stable with regard to chip movements or slight tilting during handling. The experimental results evidenced good performance for drop diameters of 3.5 mm and channel widths around 200 $\mu$m. The interconnected hanging drop system is stable and easy to handle. Initial filling can be performed rapidly, and perfusion-rates on the order of 1-50 $\mu$l/min meet most experimental requirements. Drops can be arranged in a way that is fully compatible to 384-well-plate formats.

Embodiments of the invention encompass regular arrays of different size as well as more complex networks. An important issue in using microfluidics is a bubble-free, initial filling of the channel structures, especially for multiply branched networks comprising larger reservoirs. Entrapping bubbles is rarely occurring in the open systems of embodiments of the invention, so that one can make full use of acting capillary forces. Further, the surface tension of the drops actively transports the liquid through the system until complete equilibration. The drops and their surface tension characteristics act as small, integrated pumps, so that large and complex systems can be reliably filled through a single inlet.

Commonly used microfluidic features, such as laminar flow, serial and parallel channel arrangements with channel resistance adjustment, or gradient generators can be readily implemented when using embodiments of the invention to realize functional hanging microfluidic networks as presented in the examples. In contrast to closed systems, the volumes of hanging drops, which form the liquid spaces in the network, can be changed during an experiment by adjusting the overall pressure. Further, in analogy to electric circuit models, the drops introduce capacitive elements to the network that exhibit buffer and low-pass-filter characteristics as described by Leslie et *al.* (Leslie, D. C. et al. Frequency-specific flow control in microfluidic circuits with passive elastomeric features. Nature Physics 5, 231-235 (2009)), with the liquid-air interface replacing the elastic membrane.

A hanging microfluidic network produced using embodiments of the invention is particularly suited for spherical microtissues. It has been experimentally shown that by optimizing drop and channel dimensions, bead/cell suspensions as well as loading protocols, identical-size tissue spheroids can be produced in a highly parallel approach while applying only a simple pipetting step. The microfluidic system is stable over at least a period of 4 days and can be continuously monitored on an inverted microscope. The upscaling to larger formats has been presented for the microfluidic components. The number of pipetting steps is substantially reduced also there. Perfusion of microtissues and exposure to different compounds has been experimentally presented by a continuous-flow live/dead staining. Cells were not displaced, and only low shear stresses on the microtissue surface occurred.

## Claims

1. A hanging network plate, comprising
   a network surface (12) including at least two hanging drop compartments (14) and at least one network channel (16) fluidly connecting the hanging drop compartments, wherein the at least two hanging drop compartments and the at least one network channel are surface patterns confined by at least one retention structure (18).

2. The hanging network plate of claim 1,
   wherein the hanging network plate, the at least one network channel and/or the hanging drop compartments are microfluidic; and/or
   wherein the hanging drop compartments and/or the at least one network channel form at least one micropattern; and/or
   wherein the at least one retention structure is a capillary retention structure.

3.  The hanging network plate of any of the preceding claims,
    wherein the retention structure comprises at least one element chosen from: an internal lateral edge (22) of the hanging drop compartments and/or of the network channel(s); a hydrophobic rim; a rim (23) surrounding the hanging drop compartments and/or the network channel(s);
    a rim protruding from an internal lateral edge of the hanging drop compartments and/or of the network channel(s); a retention groove (24) surrounding the hanging drop compartments and/or the network channel(s);
    a retention groove adjacent and/or surrounding the lateral edge and/or the rim; a capillary stop (26); and a combination thereof.

4.  The hanging network plate of any of the preceding claims,
    wherein the hanging drop compartments are arranged in at least one array; and/or
    wherein the at least one network channel includes a gradient generator (33); and/or
    wherein the at least one network channel and/or the hanging drop compartments include at least one guiding channel (34).

5.  The hanging network plate of claim 4,
    wherein the at least one guiding channel (34) is provided at the center of the network channels and/or of the hanging drop compartments; and/or
    wherein at least two guiding channels are provided and fluidly interconnected to each other; and/or
    wherein at least one of the hanging drop compartments has two or more fluidly interconnected guiding channels being positioned at an angle to each other; and/or
    wherein the hanging network plate comprises at least one inlet (32) for liquids, the inlet being fluidly connected to at least one of the drop compartments and/or to at least one of the network channels.

6.  The hanging network plate of claim 5,
    wherein at least one of the inlets for liquids is provided in another surface of the hanging network plate; and/or
    wherein at least one of the inlets for liquids is provided in another surface (13) of the hanging network plate, the another surface being provided diametrically opposed to the network surface.

7.  The hanging network plate of any of the preceding claims,
    wherein the hanging drop compartments and/or the network channels are grooves provided in the network surface; and/or
    wherein the at least one guiding channel is one or more surface patterns and/or one or more grooves provided in the surface of the at least one network channel and/or of the hanging drop compartments.

8.  The hanging network plate of any of the preceding claims,
    wherein the plan view shape of at least one of the hanging drop compartments is circular; and/or
    wherein at least two of the hanging drop compartments have different cross sectional sizes and/or different plan view sizes; and/or
    wherein at least two or each of the network channels have the same cross sectional size; and/or
    wherein at least two or each of the guiding channels have the same cross sectional size or different cross sectional sizes.

9.  The hanging network plate of any of the preceding claims,
    wherein the hanging drop compartments are circular and have a diameter of 2 to 7 mm, preferably 3 to 6 mm, more preferably 3.5 to 5 mm; and/or
    wherein the centers of neighboring hanging drop compartments have a distance of 2.5 to 30 mm, preferably 3 to 10 mm, more preferably 4.5 to 9 mm, from each other, wherein the hanging drop compartments have a depth of 0.1 to 2 mm, preferably 0.2 to 1.5 mm; and/or
    wherein the network channels have a width of 0.1 to 1.0 mm, preferably 0.1 to 0.4 mm, more preferably 0.2 mm; and/or
    wherein the network channels have a length of 0.05 to 5 cm, preferably 0.2 to 4 cm;
    wherein the network channels have a depth of 0.1 to 2 mm,
    preferably 0.2 to 1.8 cm; and/or
    wherein the guiding channels have a width of 0.1 to 1 mm, preferably 0.2 to 0.8 mm; and/or
    wherein the guiding channels have a depth of 0.1 to 0.5 mm, preferably 0.2 to 0.4 mm.

10. The hanging network plate of any of the preceding claims, wherein the hanging network plate is rigid and/or the surface patterns and/or retention structures are of a soft material; and/or wherein the hanging network plate comprises a material chosen from polymer and/or metal, in particular silicone or poly(dimethylsiloxane); and/or wherein the hanging network plate is produced by casting polymer materials from a negative mold, injection molding and/or structuring by a (hot)embossing process and/or by structuring polymers and/or metals using machining, milling, shape cutting, and/or chipping technology.

11. A method for producing a hanging network plate of any of
the preceding claims, comprising
forming a hanging network plate (10) having a network surface (12) including at least two hanging drop compartments (14) and at least one network channel (16) fluidly connecting the hanging drop compartments, wherein the at least two hanging drop compartments and the at least one network channel are surface patterns confined by at least one retention structure (18); and/or
forming a negative mold of the hanging network plate (10) and casting the material of the hanging network plate in the mold.

12. The method of claim 11,
wherein the negative mold is silanized before casting; and/or
wherein holes are provided in the plate at the inlet positions; and/or
wherein the casted plate is cleaned or selectively cleaned and/or treated or selectively treated with oxygen plasma; and/or
wherein the network surface and/or the retention structure of the casted plate is covered with a thin layer of a hydrophobic material and/or of the material of the plate except for the hanging drop compartments (14) and the at least one network channel (16)); and/or wherein the retention structure is made or kept hydrophobic while the hanging drop compartments and channels are made or kept hydrophilic ; and/or
wherein the retention structure is made or kept hydrophilic while the hanging drop compartments and channels are made or kept hydrophobic.

13. A use of the hanging network plate of any of the
preceding claims for producing and/or culturing cells or tissue, for engineering tissue, for forming spherical microtissue and/or cell aggregates, for performing assays, for performing in-vitro cell-based assays, for performing toxicity assays, for performing protein crystallization, and/or for forming material aggregates.

14. A method for producing a hanging liquid network including
at least one liquid drop and/or a liquid network, comprising
arranging the hanging network plate of any of claims 1 to 13 such that the network surface is positioned at the bottom of the hanging network plate.

15. The method of claim 14, comprising
introducing at least one liquid into at least one of the inlets and/or removing at least one liquid, wherein at least one of the liquids comprises a suspension of cells, tissue, proteins, and/or another compound interfering with the cells, tissue and/or proteins.

16. The method of claim 14 or 15, comprising
perfusing the hanging liquid network with liquid, in particular for maintaining a culturing condition; and/or exposing matter in the at least one liquid drop to a compound; and/or
removing liquid from the liquid drop and/or liquid network for analysis.

FIG 1

FIG 2a

FIG 2b

FIG 3a

FIG 3b

FIG 3c

FIG 3d

FIG 4a

FIG 4b

# FIG 5a

# FIG 5b

FIG 5c

FIG 5d

FIG 5e

FIG 5f

FIG 6a

FIG 6b

FIG 7

A

t$_0$    t$_0$+10 min    t$_0$+20 min    t$_0$+30 min    t$_0$+24h

B

1    2    3    4    5

100 µm

C

5 mm

FIG 8

A

t = 1 h    18 h    36 h    54 h    72 h

B

a    b    c    d    e

t = 90 h

200 µm

# FIG 9

$Q_{in} = Q_{out} = 2\ \mu l/min$

# FIG 10

Europäisches
Patentamt

European
Patent Office

Office européen
des brevets

# EUROPEAN SEARCH REPORT

Application Number

EP 13 17 1049

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/287470 A1 (STOPPINI LUC [CH]) 24 November 2011 (2011-11-24) * paragraph [0074] - paragraph [0084] * * paragraph [0103] - paragraph [0106] * * paragraph [0146] * * paragraph [0154] * * figures 1A-1C, 4A, 9B * | 1-16 | INV. C12M3/06 C12M1/12 |
| A | WO 2012/117083 A2 (HOFFMANN LA ROCHE [CH]; FATTINGER CHRISTOF [CH]; IAIZA PATRICK [FR]; K) 7 September 2012 (2012-09-07) * claims 1-15; figures 6-9 * | 1-16 | |
| A | US 2013/084634 A1 (HSU CHIA-HSIEN [TW] ET AL) 4 April 2013 (2013-04-04) * paragraph [0052] - paragraph [0061] * * paragraph [0133] - paragraph [0136] * * figures 1A-1D,3B,16 * | 1-16 | |

| | |
|---|---|
| | TECHNICAL FIELDS SEARCHED (IPC) |
| | C12M C12N B01L |

The present search report has been drawn up for all claims

| Place of search | Date of completion of the search | Examiner |
|---|---|---|
| Berlin | 17 September 2013 | Cubas Alcaraz, Jose |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                                EP 13 17 1049

This annex lists the patent family members relating to the patent documents cited in  the above-mentioned European search report.
The members are as contained in the European Patent Office EDP file on
The European Patent Office is in no way liable for these particulars which are merely given for the purpose of information.

17-09-2013

| Patent document cited in search report | | Publication date | Patent family member(s) | | Publication date |
|---|---|---|---|---|---|
| US 2011287470 | A1 | 24-11-2011 | AU | 2009283944 A1 | 25-02-2010 |
|  |  |  | CA | 2769282 A1 | 25-02-2010 |
|  |  |  | EP | 2376624 A2 | 19-10-2011 |
|  |  |  | NZ | 591812 A | 31-08-2012 |
|  |  |  | US | 2011287470 A1 | 24-11-2011 |
|  |  |  | WO | 2010020876 A2 | 25-02-2010 |
| WO 2012117083 | A2 | 07-09-2012 | NONE | | |
| US 2013084634 | A1 | 04-04-2013 | US | 2013084634 A1 | 04-04-2013 |
|  |  |  | WO | 2013049165 A1 | 04-04-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Biotechnology & genetic engineering reviews,* 2010, vol. 26, 117-38
- **LEE, J. ; CUDDIHY, M. J. ; KOTOV, N.** Three- dimensional cell culture matrices: state of the art. Tissue engineering. Part B. *Three-dimensional cell culture matrices: state of the art. Tissue engineering. Part B,* 2008, vol. 14, 61-86
- **KHADEMHOSSEINI, A. ; LANGER, R. ; BORENSTEIN, J. ; VACANTI, J. P.** Microscale technologies for tissue engineering and biology. *Proceedings of the National Academy of Sciences of the United States of America,* 2006, vol. 103, 2480-7
- **HUH, D. ; TORISAWA, Y. ; HAMILTON, G. A, KIM, H. J. ; INGBER, D. E.** Microengineered physiological biomimicry: organs-on-chips. *Lab on a chip,* 2012, vol. 12, 2156-64
- **FRIEDRICH, J. ; EBNER, R. ; KUNZ-SCHUGHART, L.** a Experimental anti-tumor therapy in 3-D: spheroids - old hat or new challenge?. *International journal of radiation biology,* 2007, vol. 83, 849-71
- **HIRSCHHAEUSER, F. et al.** Multicellular tumor spheroids: an underestimated tool is catching up again. *Journal of biotechnology,* 2010, vol. 148, 3-15
- **KELM, J. M. ; FUSSENEGGER, M.** Microscale tissue engineering using gravity-enforced cell assembly. *Trends in biotechnology,* 2004, vol. 22, 195-202
- **HSIAO, A. Y. et al.** Micro-ring structures stabilize microdroplets to enable long term spheroid culture in 384 hanging drop array plates. *Biomedical microdevices,* 2012, vol. 14, 313-23
- **DREWITZ, M. et al.** Towards automated production and drug sensitivity testing using scaffold-free spherical tumor microtissues. *Biotechnology journal,* 2011, vol. 6, 1488-96
- **ESCH, M. B. ; KING, T. L. ; SHULER, M. L.** The Role of Body-on-a-Chip Devices in Drug and Toxicity Studies. *Annual review of biomedical engineering,* 2011, vol. 13, 55-72
- **HUH, D. ; HAMILTON, G. A. ; INGBER, D. E.** From 3D cell culture to organs-on-chips. *Trends in cell biology,* 2011, vol. 21, 745-54
- **JEON, N. L. et al.** Generation of Solution and Surface Gradients Using Microfluidic Systems. *Langmuir,* 2000, vol. 16, 8311-8316
- **LESLIE, D. C. et al.** Frequency-specific flow control in microfluidic circuits with passive elastomeric features. *Nature Physics,* 2009, vol. 5, 231-235